# EUROPEAN PATENT SPECIFICATION

(11) **EP 2 777 649 B1**
(45) Date of publication and mention of the grant of the patent: **05.07.2017**
(21) Application number: 14159030.7
(22) Date of filing: 12.03.2014
(51) Int. Cl.: A61F 2/966, A61F 2/848

(54) **Delivery system for expandable stents**
Abgabesystem für expandierbare Stents
Système d'administration pour des endoprothèses extensibles

(30) Priority: 13.03.2013 US 201313801905
(43) Date of publication of application: 17.09.2014
(60) Divisional application: 16189783.0
(73) Proprietor: DePuy Synthes Products, LLC, Raynham, MA 02767 (US)
(72) Inventor: Girnary, Hussein H., Miami, FL 33130 (US); Sotodelvalle, Ariel, Hialeah, FL 33015 (US); Lorenzo, Juan A., Davie, FL 33328 (US); Forsythe, Peter, Miami, FL 33130 (US)
(74) Representative: Carpmaels & Ransford LLP

(56) References cited:
- EP-A1- 1 400 219
- US-A1- 2007 255 385
- US-A1- 2009 306 761
- US-A1- 2011 301 690

## Description

### BACKGROUND OF THE INVENTION

This invention relates generally to devices for interventional therapeutic treatment or vascular surgery for treatment of defects in the vasculature, and more particularly concerns a system for delivering a self-expanding stent to a treatment site in a vasculature of a patient.

Stents, which are tubular reinforcements inserted into a blood vessel to provide an open path within the blood vessel, have been widely used in intravascular angioplasty treatment of occluded cardiac arteries. In such applications, the stent is inserted after an angioplasty procedure or the like in order to prevent restenosis of the artery. In these applications, the stents are often deployed by use of inflatable balloons, or mechanical devices which force the stent open, thereby reinforcing the artery wall and provide a clear through-path in the center of the artery after the angioplasty procedure to prevent restenosis.

While such procedures may be useful in certain aspects of vascular surgery in which vasoocclusive devices are used, the weakness of the vasculature and the tortuosity of the neurovasculature places limits on the applicability of such stents in procedures to repair neurovascular defects. Furthermore, the use of placement techniques, such as balloons or mechanical expansions of the type often found to be useful in cardiac surgery, are relatively less useful in vasoocclusive surgery, particularly when tiny vessels, such as those found in the brain, are to be treated. Hence, those skilled in the art have recognized a need for a stent compatible with techniques in vasoocclusive treatment of neurovascular defects that provides selective reinforcement in the vicinity of a neurovascular defect, while avoiding any unnecessary trauma or risk of rupture to the blood vessel.

An expandable stent and delivery system is known that includes an expandable stent having proximal and distal anchor members mounted on proximal and distal legs extending proximally and distally from the stent. The proximal and distal anchor members of the expandable stent are mounted in gaps formed between proximal, intermediate and distal cylindrical members disposed on and spaced apart along an elongated core member. However, pushing the device distally in a catheter from the proximal end of the device is not optimal, because application of force in a distal direction on the proximal end of the stent can axially compress the stent, and can cause the stent to expand radially. Likewise, retracting the device proximally may not be optimal either, because application of force in a proximal direction on the distal end of the stent also can axially compress the stent, and can cause the stent to expand radially.

EP1400219 A1 discloses an expandable stent and a delivery system (12), for enhancing luminal dilation of a blood vessel and treating aneurysms, including proximal, intermediate and distal cylindrical members disposed on and spaced apart along an elongated core member such that first (36) and second gaps are formed. The expandable stent includes anchor members which align with the gaps. The expandable stent is mounted on the intermediate cylindrical member, and the anchor members are disposed within the gaps thereby locking the stent onto the core member.

US2007255385A1 describes an expandable stent and delivery system for enhancing luminal dilation of a blood vessel and treating aneurysms, and includes proximal, intermediate and distal cylindrical members disposed on and spaced apart along an elongated core member so that first and second gaps are formed. The expandable stent includes anchor members which align with the gaps. The expandable stent is mounted on the intermediate cylindrical member, and the anchor members are disposed within the gaps thereby locking the stent onto the core member.

Itwould be desirable to provide a delivery system for expandable stents that offers the flexibility of engaging the device at a desired proximal, distal or other location anywhere in between, for pushing and/orpulling the device proximally or distally as desired. The present invention meets these and otherneeds.

### SUMMARY OF THE INVENTION

Methods of using the device are presented as a way to understand the invention and do not form part of the invention. Briefly and in general terms, the present invention provides for a system for delivering and releasing a self-expanding stent to a treatment site in a patient's vasculature that allows the self-expanding stent to fit and move more easily within a constrained space in a catheter, by reducing localized buckling and radial expansion of the self-expanding stent. The system includes features for moving the self-expanding stent back and/or forth inside a catheter preferentially from one or more of a proximal end, distal end or some other specific location therebetween of the self-expanding stent, essentially by allowing the self-expanding stent to be moved distally and proximally by dragging or pulling the self-expanding stent from distal and/or proximal portions, respectively, instead of by pushing the proximal end of the self-expanding stent distally or pushing the distal end of the self-expanding stent proximally, and thereby reducing the force needed to drive the device forward distally and/or rearwardly proximally. The present invention is defined by the independent claim. Further details of specific embodiments are given in the dependent claims.

Other features and advantages of the present invention will become more apparent from the following detailed description of the preferred embodiments in conjunction with the accompanying drawings, which illustrate, by way of example, the operation of the invention.

### BRIEF DESCRIPTION OF THE DRAWINGS

Figure 1 is an enlarged partial cross-sectional schematic diagram of a system for delivering and releasing a self-expanding stent to a treatment site in a patient's vasculature, according to the invention.
Fig. 2 is an enlarged partial cross-sectional schematic diagram similar to Fig. 1, showing the system disposed within a blood vessel and aligned adjacent to a treatment site in a patient's vasculature.
Fig. 3 is an enlarged partial cross-sectional schematic diagram similar to Fig. 2, with a portion of the self-expanding stent cut away to expose radiopaque markers or stop members on the core advancement wire.
Fig. 4 is an enlarged partial cross-sectional schematic diagram similar to Fig. 3, illustrating pushing the device distally.
Fig. 5 is an enlarged partial cross-sectional schematic diagram similar to Fig. 4, illustrating pulling the device proximally.
Fig. 6 is an enlarged partial sectional view of a deployment catheter moved proximally with a proximal portion of the self-expanding stent compressed within a deployment catheter and a distal portion of the self-expanding stent expanded within the patient's vasculature.
Fig. 7 is a cross-sectional elevational schematic diagram illustrating an expanded configuration of the self-expanding stent of Fig. 1 within the patient's vasculature.
Fig. 8 is a cross-sectional elevational schematic diagram of a variation of the system of Fig. 1, with a portion of the self-expanding stent cut away to expose radiopaque markers or stop members on the core advancement wire.
Fig. 9 is an enlarged partial sectional view of the self-expanding stent and delivery system disposed within a patient's vasculature and aligned adjacent to an aneurysm.
Fig. 10 is an enlarged partial sectional view of the deployment catheter moved proximally, with the proximal portion of the self-expanding stent constrained within the deployment catheter and the distal portion of the self-expanding stent expanded within the patient's vasculature.
Fig. 11 is an enlarged sectional view of the self-expanding stent expanded within the patient's vasculature and covering a mouth of the aneurysm.
Fig. 12 is an enlarged sectional view of the stent expanded within the vessel and a microcatheter inserted through the wall of the self-expanding stent and into the aneurysm.
Fig. 13 is an enlarged sectional view of the self-expanding stent expanded within the patient's vasculature and covering the mouth of the aneurysm with an embolic coil deployed within the aneurysm.

### DETAILED DESCRIPTION OF THE PREFERRED EMBODIMENTS

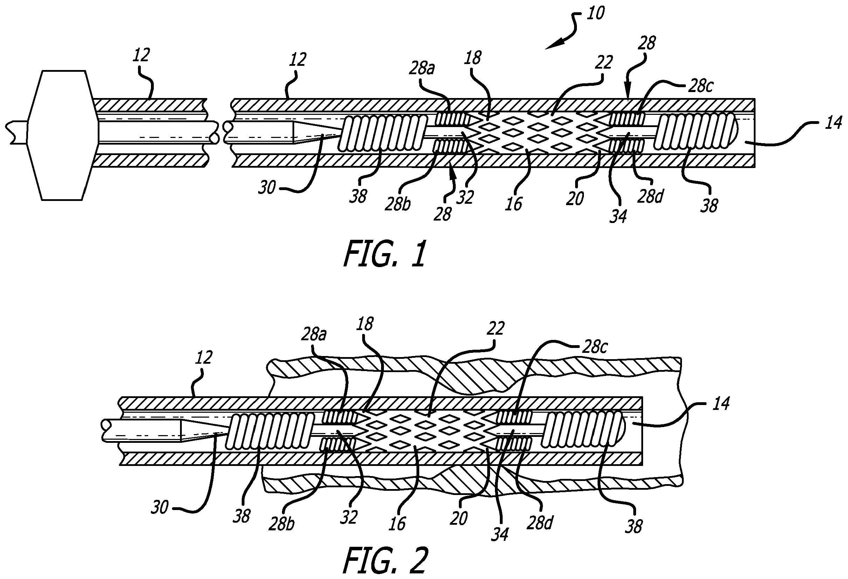

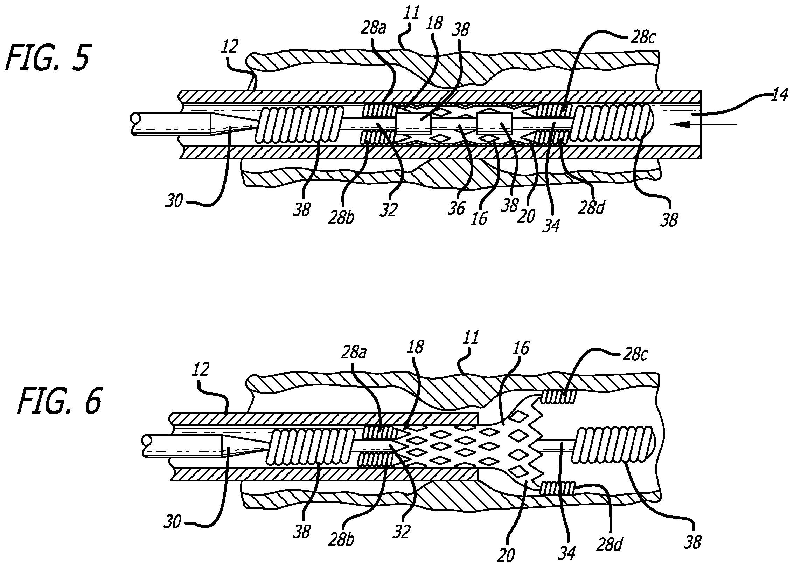

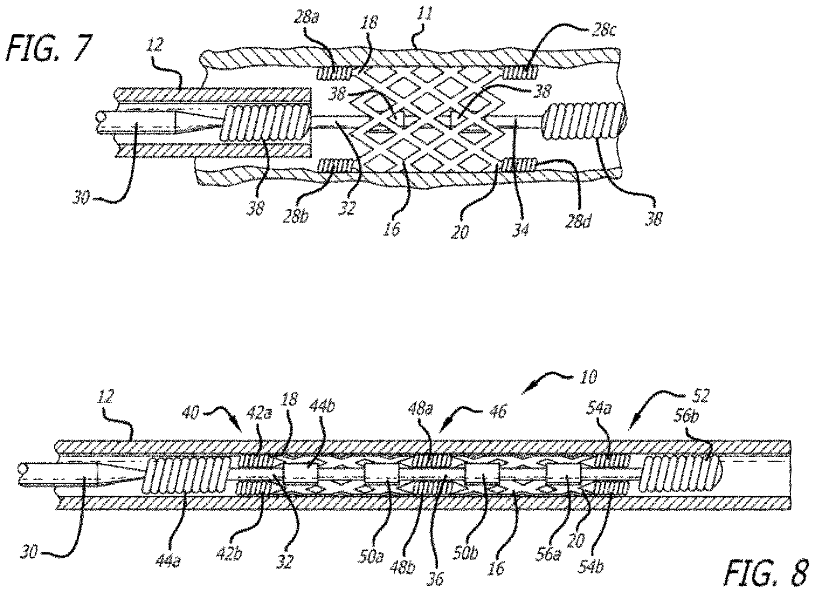

Referring to the drawings, which are provided by way of example, and not by way of limitation, the present invention provides for an apparatus 10 for delivering and releasing a self-expanding stent to a treatment site in a patient's vasculature 11. As is illustrated in Fig. 1, the apparatus includes a catheter 12 having an inner lumen 14, a tubular self-expanding stent 16 having an inner lumen (not shown), a proximal end 18, a distal end 20, and an intermediate portion 22 located between the proximal end and the distal end, and an inner lumen (not shown). Referring to Figs. 1-5, the tubular self-expanding stent has a compressed configuration dimensioned to fit within the inner lumen of the catheter, such that the tubular self-expanding stent is configured to be constrained from expanding when the tubular self-expanding stent contained within the catheter, and an expanded configuration, illustrated in Fig. 7. The tubular self-expanding stent includes one or more anchor members 28, such as proximal anchor members 28a, 28b disposed on opposing sides of the core advancement wire, for example, at the proximal end of the tubular self-expanding stent, and distal anchor members 28c, 28d disposed on opposing sides of the core advancement wire, for example, at the distal end of the tubular self-expanding stent, connected to and extending radially inwardly from the tubular self-expanding stent, as will be explained further below.

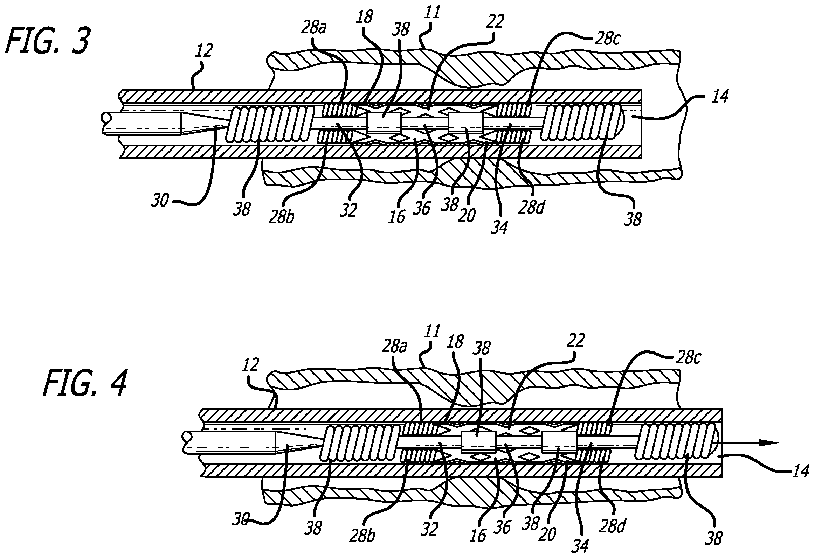

A core advancement wire 30 is disposed within and extends through the lumen of the tubular stent. The core advancement wire has a proximal portion 32, a distal portion 34, and an intermediate portion 36 (shown in Figs. 3-5 and 7) located between the proximal and distal portions of the core advancement wire. The core advancement wire advantageously includes one or more radiopaque markers or stop members 38 extending radially outwardly from the core advancement wire and configured to engage the one or more anchor members when the core advancement wire is translated longitudinally toward the one or more anchor members, whereby force applied longitudinally to the core advancement wire is transmitted through the one or more anchor members to the tubular self-expanding stent and acts to move the tubular self-expanding stent through the catheter when the stent is constrained within the catheter. In a presently preferred aspect, the one or more radiopaque markers or stop members have a diameter greater than or equal to an inward radial extension of the one or more anchor members when the tubular self-expanding stent is in the compressed configuration.

In one presently preferred aspect, the one or more radiopaque markers or stop members can be formed as a sleeve or a coil made of polymer or metal, for example, and fixedly attached to the core advancement wire. In another presently preferred aspect, the one or more radiopaque markers or stop members can be formed as a ground step profile formed on the core advancement wire, or as an enlarged portion of the core advancement wire having a diameter greater than or equal to an inward radial extension of the one or more anchor members when the tubular self-expanding stent is in the compressed configuration.

Referring to Fig. 8, the one or more anchor members includes one or more proximal anchor members 40, such as two proximal anchor members 42a, 42b disposed on opposing sides of the core advancement wire, for example, and disposed at or near the proximal end of the tubular self-expanding stent, extending radially outwardly of the core advancement wire. The one or more proximal anchor members are configured to be engaged by the one or more proximal radiopaque markers or stop members, such as first and second proximal radiopaque markers or stop members 44a, 44b spaced apart from each other, with the one or more proximal anchor members disposed between the first and second proximal radiopaque markers or stop members.

According to the invention, the one or more anchor members also include one or more intermediate anchor members 46, such as two intermediate anchor members 48a, 48b disposed on opposing sides of the core advancement wire, for example, and disposed at or near the intermediate portion of the tubular self-expanding stent, extending radially outwardly of the core advancement wire and configured to be engaged by the one or more intermediate radiopaque markers or stop members, such as first and second intermediate radiopaque markers or stop members 50a, 50b spaced apart from each other, with the one or more intermediate anchor member disposed between the first and second intermediate radiopaque markers or stop members.

The one or more anchor members also include one or more distal anchor members 52, such as two distal anchor members 54a, 54b disposed on opposing sides of the core advancement wire, for example, and disposed at or near the distal end of the tubular self-expanding stent, extending radially outwardly of the core advancement wire. The one or more distal anchor members are configured to be engaged by one or more distal radiopaque markers or stop members, such as first and second distal radiopaque markers or stop members 56a, 56b spaced apart from each other, with the one or more distal anchor members disposed between the first and second distal radiopaque markers or stop members, when the core advancement wire is translated longitudinally toward the one or more distal anchor members.

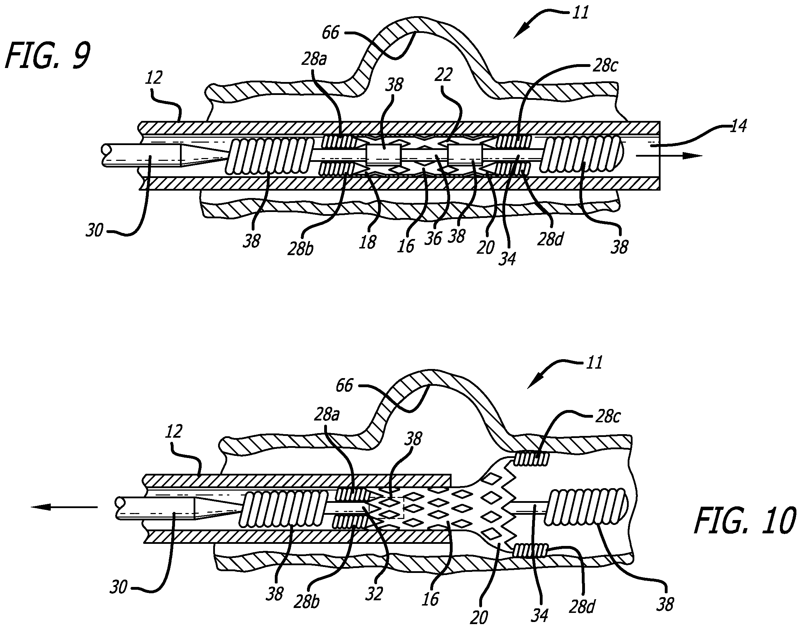

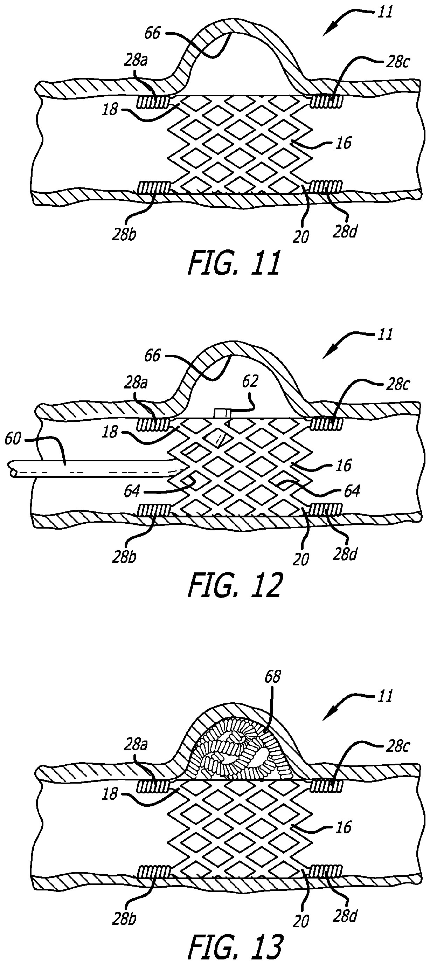

Referring to Fig. 9, the catheter and self-expanding stent can, for example, be positioned at a treatment site within a patient's vasculature and aligned across a mouth of an aneurysm 66. As is shown in The self-expanding stent can be at first only partially deployed within the patient's vasculature, affording the opportunity of retracting the self-expanding stent proximally within the catheter, as is illustrated in Fig. 10, such as for later repositioning and deployment of the self-expanding stent. The self-expanding stent can be fully deployed by moving the self-expanding stent distally and withdrawing the catheter proximally, causing the anchor members on the distal end of the self-expanding stent to anchor the distal end of the self-expanding stent to allow the self-expanding stent to deploy to cover the mouth of the aneurysm, as is illustrated in Fig. 11.

Referring to Fig. 12, once the self-expanding stent is deployed cover the mouth of the aneurysm, a microcatheter 60 can be inserted into the patient's vasculature, and a distal portion 62 of the microcatheter may be insert through one of the interstices 64 of the self-expanding stent, and into the aneurysm, so that one or more embolic coils 68 or other embolic agents can be delivered into the aneurysm, as is illustrated in Fig. 13.

It will be apparent from the foregoing that while particular forms of the invention have been illustrated and described, various modifications can be made without departing from the invention. Accordingly, it is not intended that the invention be limited, except as by the appended claims.

## Claims

1. A system for delivering and releasing a self-expanding stent to a treatment site in a patient's vasculature, said system comprising:
a catheter (12) having an inner lumen;
a tubular self-expanding stent (16) having an inner lumen, a proximal end (18), a distal end (20), and an intermediate portion located between said proximal end and said distal end, and an inner lumen, said tubular self-expanding stent having a compressed configuration dimensioned to fit within said inner lumen of said catheter and an expanded configuration, said tubular self-expanding stent being configured to be constrained from expanding when said tubular self-expanding stent contained within said catheter, and said tubular self-expanding stent including at least one anchor member (42,48,54) extending radially inwardly into said inner lumen; and
a core advancement wire (30) disposed within and extending through said lumen of said tubular stent, said core advancement wire having a proximal portion (32), a distal portion (34), an intermediate portion (36) located between said proximal and distal portions of said core advancement wire, and said core advancement wire having at least one stop member (44,50,56) extending radially outwardly from said core advancement wire and configured to engage said at least one anchor member when said core advancement wire is translated longitudinally toward said at least one anchor member, whereby force applied longitudinally to said core advancement wire is transmitted through said at least anchor member to said tubular self-expanding stent and acts to move said tubular self-expanding stent through said catheter when said stent is constrained within said catheter
wherein said at least one anchor member comprises at least one distal anchor member (52) disposed at or near said distal end of said tubular self-expanding stent, and said at least one stop member comprises at least one distal stop member (56a) extending radially outwardly from said core advancement wire and configured to engage said at least one distal anchor member when said core advancement wire is translated distally longitudinally toward said at least one distal anchor member,
**characterized in that** said at least one anchor member comprises at least one intermediate anchor member (46) disposed at or near said intermediate portion of said tubular self-expanding stent, and said at least one stop member comprises at least one intermediate stop member (50a,50b) extending radially outwardly from said core advancement wire and configured to engage said at least one intermediate anchor member when said core advancement wire is translated longitudinally toward said at least one intermediate anchor member.

2. The system of Claim 1, wherein said at least one anchor member comprises at least one proximal anchor member (40) disposed at or near said proximal end of said tubular self-expanding stent, and said at least one stop member comprises at least one proximal stop member extending radially outwardly from said core advancement wire and configured to engage said at least one proximal anchor member when said core advancement wire is translated longitudinally toward said at least one proximal anchor member.

3. The system of Claim 2, wherein said at least one proximal anchor member comprises two proximal anchor members (42a,42b) disposed on opposing sides of said core advancement wire.

4. The system of Claim 2, wherein said at least one stop member comprises first and second proximal stop members (44a,44b) spaced apart from each other, and said at least one proximal anchor member is disposed between said first and second proximal stop members.

5. The system of Claim 3, wherein said at least one stop member (38) comprises first and second proximal stop members (44a,44b) spaced apart from each other, and said two proximal anchor members are disposed between said first and second proximal stop members.

6. The system of Claim 1, wherein said at least one distal anchor member comprises two distal anchor members (54a,54b) disposed on opposing sides of said core advancement wire.

7. The system of Claim 1, wherein said at least one stop member comprises first and second distal stop members (56a,56b) spaced apart from each other, and said at least one distal anchor member (52) is disposed between said first and second distal stop members.

8. The system of Claim 6, wherein said at least one stop member comprises first and second distal stop members (56a,56b) spaced apart from each other, and said two distal anchor members are disposed between said first and second distal stop members.

9. The system of Claim 1, wherein said at least one intermediate anchor member comprises two intermediate anchor members (48a,48b) disposed on opposing sides of said core advancement wire.

10. The system of Claim 1, wherein said at least one stop member comprises first and second intermediate stop members (50a,50b) spaced apart from each other, and said at least one intermediate anchor member is disposed between said first and second intermediate stop members.

11. The system of Claim 9, wherein said at least one stop member comprises first and second intermediate stop members (50a,50b) spaced apart from each other, and said two intermediate anchor members are disposed between said first and second intermediate stop members.

12. The system of Claim 1, wherein said at least one stop member (44,50,56) has a diameter greater than or equal to an inward radial extension of said at least one anchor member when said tubular self-expanding stent is in said compressed configuration.

13. The system of Claim 1, wherein said at least one stop member (44,50,56) comprises a sleeve fixedly attached to said core advancement wire, a coil fixedly attached to said core advancement wire, or a ground step profile formed on said core advancement wire.

14. The system of Claim 1, wherein said at least one stop member (40,50,56) comprises an enlarged portion of said core advancement wire having a diameter greater than or equal to an inward radial extension of said at least one anchor member when said tubular self-expanding stent is in said compressed configuration.

## Patentansprüche

1. System zur Abgabe und Freisetzung eines selbstexpandierenden Stents an eine Behandlungsstelle in die Blutgefäße eines Patienten, wobei das System Folgendes umfasst:
einen Katheter (12), der ein inneres Lumen aufweist;
einen röhrenförmigen selbstexpandierenden Stent (16), der ein inneres Lumen aufweist, ein proximales Ende (18), ein distales Ende (20), und einen Zwischenteil, der sich zwischen dem proximalen und dem distalen Ende befindet, und ein inneres Lumen, wobei der röhrenförmige selbstexpandierende Stent eine komprimierte Konfiguration, die dimensioniert ist, um in das innere Lumen des Katheters zu passen, und eine expandierte Konfiguration aufweist, wobei der röhrenförmige selbstexpandierende Stent konfiguriert ist, um am Expandieren gehindert zu werden, wenn der röhrenförmige selbstexpandierende Stent in dem Katheter enthalten ist, und der röhrenförmige selbstexpandierende Stent mindestens ein Verankerungselement (42, 48, 54) umfasst, das sich radial nach innen in das innere Lumen erstreckt; und
einen Führungsdraht (30) der innerhalb des Lumens des röhrenförmigen Stents angeordnet ist und sich durch dieses erstreckt, wobei der Führungsdraht einen proximalen Teil (32), einen distalen Teil (34), einen Zwischenteil (36) aufweist, der sich zwischen dem proximalen und dem distalen Teil des Führungsdrahts befindet, und wobei der Führungsdraht mindestens ein Halteelement (44, 50, 56) aufweist, das sich radial nach außen von dem Führungsdraht erstreckt und konfiguriert ist, um in das mindestens eine Verankerungselement einzugreifen, wenn der Führungsdraht in der Längsrichtung zu dem mindestens einen Verankerungselement verschoben wird, wobei Kraft, die in der Längsrichtung auf den Führungsdraht ausgeübt wird, durch das mindestens eine Verankerungselement auf den röhrenförmigen selbstexpandierenden Stent übertragen wird und wirkt, um den röhrenförmigen selbstexpandierenden Stent durch den Katheter zu bewegen, wenn der Stent in dem Katheter eingeschlossen ist,
wobei das mindestens eine Verankerungselement mindestens ein distales Verankerungselement (52) aufweist, das an oder in der Nähe des distalen Endes des röhrenförmigen selbstexpandierenden Stents angeordnet ist, und das mindestens eine Halteelement mindestens ein distales Halteelement (56a) umfasst, das sich radial nach außen von dem Führungsdraht erstreckt und konfiguriert ist, um in das mindestens eine distale Verankerungselement einzugreifen, wenn der Führungsdraht distal in der Längsrichtung zu dem mindestens einen distalen Verankerungselement verschoben wird,
**dadurch gekennzeichnet, dass** mindestens ein Verankerungselement mindestens ein Zwischenverankerungselement (46) aufweist, das an dem oder in der Nähe des Zwischenteil(s) des röhrenförmigen selbstexpandierenden Stents angeordnet ist, und das mindestens eine Halteelement mindestens ein Zwischenhalteelement (50a, 50b) umfasst, das sich radial nach außen von dem Führungsdraht erstreckt und konfiguriert ist, um in das mindestens eine Zwischenverankerungselement einzugreifen, wenn der Führungsdraht in der Längsrichtung zu dem mindestens einen Zwischenverankerungselement verschoben wird.

2. System nach Anspruch 1, wobei das mindestens eine Verankerungselement mindestens ein proximales Verankerungselement (40) umfasst, das an dem oder in der Nähe des proximalen Ende(s) des röhrenförmigen selbstexpandierenden Stents angeordnet ist, und das mindestens eine Halteelement mindestens ein proximales Halteelement umfasst, das sich radial nach außen von dem Führungsdraht erstreckt und konfiguriert ist, um in mindestens ein proximales Verankerungselement einzugreifen, wenn der Führungsdraht in der Längsrichtung zu dem mindestens einen proximalen Verankerungselement verschoben wird.

3. System nach Anspruch 2, wobei das mindestens eine proximale Verankerungselement zwei proximale Verankerungselemente (42a, 42b) umfasst, die auf entgegengesetzten Seiten des Führungsdrahts angeordnet sind.

4. System nach Anspruch 2, wobei das mindestens eine Halteelement ein erstes und ein zweites proximales Halteelement (44a, 44b) umfasst, die in einem Abstand zueinander angeordnet sind, und das mindestens eine proximale Verankerungselement zwischen dem ersten und dem zweiten proximalen Halteelement angeordnet ist.

5. System nach Anspruch 3, wobei das mindestens eine Halteelement (38) ein erstes und ein zweites proximales Halteelement (44a, 44b) umfasst, die in einem Abstand zueinander angeordnet sind, und die zwei proximalen Verankerungselemente zwischen dem ersten und dem zweiten proximalen Halteelement angeordnet sind.

6. System nach Anspruch 1, wobei das mindestens eine distale Verankerungselement zwei distale Verankerungselemente (54a, 54b) umfasst, die auf entgegengesetzten Seiten des Führungsdrahts angeordnet sind.

7. System nach Anspruch 1, wobei das mindestens eine Halteelement ein erstes und ein zweites distales Halteelement (56a, 56b) umfasst, die in einem Abstand zueinander angeordnet sind, und das mindestens eine distale Verankerungselement (52) zwischen dem ersten und dem zweiten distalen Halteelement angeordnet ist.

8. System nach Anspruch 6, wobei das mindestens eine Halteelement ein erstes und ein zweites distales Halteelement (56a, 56b) umfasst, die in einem Abstand zueinander angeordnet sind, und die zwei distalen Verankerungselemente zwischen dem ersten und dem zweiten distalen Halteelement angeordnet sind.

9. System nach Anspruch 1, wobei das mindestens eine Zwischenverankerungselement zwei Zwischenverankerungselemente (48a, 48b) umfasst, die auf entgegengesetzten Seiten des Führungsdrahtes angeordnet sind.

10. System nach Anspruch 1, wobei das mindestens eine Halteelement ein erstes und ein zweites Zwischenhalteelement (50a, 50b) umfasst, die in einem Abstand zueinander angeordnet sind, und das mindestens eine Zwischenverankerungselement zwischen dem ersten und dem zweiten Zwischenhalteelement angeordnet ist.

11. System nach Anspruch 9, wobei das mindestens eine Halteelement ein erstes und ein zweites Zwischenhalteelement (50a, 50b) umfasst, die in einem Abstand zueinander angeordnet sind, und die zwei Zwischenverankerungselemente zwischen dem ersten und dem zweiten Zwischenhalteelement angeordnet sind.

12. System nach Anspruch 1, wobei das mindestens eine Halteelement (44, 50, 56) einen Durchmesser aufweist, der größer als oder gleich eine(r) innere(n) radiale(n) Verlängerung des mindestens einen Verankerungselements ist, wenn der röhrenförmige selbstexpandierende Stent in der komprimierten Konfiguration ist.

13. System nach Anspruch 1, wobei das mindestens eine Halteelement (44, 50, 56) eine Hülse umfasst, die fest an dem Führungsdraht befestigt ist, eine Spule, die fest an dem Führungsdraht befestigt ist, oder ein Stufenprofil, das auf dem Führungsdraht gebildet wird.

14. System nach Anspruch 1, wobei das mindestens eine Halteelement (40, 50, 56) einen vergrößerten Teil des Führungsdrahts umfasst, der einen Durchmesser aufweist, der größer als oder gleich eine(r) innere(n) radiale(n) Verlängerung des mindestens einen Verankerungselements ist, wenn der röhrenförmige selbstexpandierende Stent in der komprimierten Konfiguration ist.

## Revendications

1. Système de pose et de libération d'une endoprothèse auto-extensible sur un site de traitement dans le système vasculaire d'un patient, ledit système comprenant:
un cathéter (12) présentant une lumière intérieure;
une endoprothèse auto-extensible tubulaire (16) présentant une lumière intérieure, une extrémité proximale (18), une extrémité distale (20), et une partie intermédiaire située entre ladite extrémité proximale et ladite extrémité distale, et une lumière intérieure, ladite endoprothèse auto-extensible tubulaire présentant une configuration comprimée dimensionnée de manière à s'agencer à l'intérieur de ladite lumière intérieure dudit cathéter et une configuration étendue, ladite endoprothèse auto-extensible tubulaire étant configurée de manière à être empêchée de s'étendre lorsque ladite endoprothèse auto-extensible tubulaire est contenue à l'intérieur dudit cathéter, et ladite endoprothèse auto-extensible tubulaire comprenant au moins un élément d'ancrage (42, 48, 54) qui s'étend radialement vers l'intérieur dans ladite lumière intérieure; et
un fil d'avancement de noyau (30) disposé à l'intérieur et s'étendant à travers ladite lumière de ladite endoprothèse tubulaire, ledit fil d'avancement de noyau présentant une partie proximale (32), une partie distale (34), une partie intermédiaire (36) située entre lesdites parties proximale et distale dudit fil d'avancement de noyau, et ledit fil d'avancement de noyau présentant au moins un élément d'arrêt (44, 50, 56) s'étendant radialement vers l'extérieur à partir dudit fil d'avancement de noyau et configuré de manière à engager ledit au moins un élément d'ancrage lorsque ledit fil d'avancement de noyau est déplacé de façon longitudinale en direction dudit au moins un élément d'ancrage, moyennant quoi une force appliquée de façon longitudinale audit fil d'avancement de noyau est transmise à travers ledit au moins un élément d'ancrage à ladite endoprothèse auto-extensible tubulaire et agit pour déplacer ladite endoprothèse auto-extensible tubulaire à travers ledit cathéter lorsque ladite endoprothèse est contrainte à l'intérieur dudit cathéter,
dans lequel ledit au moins un élément d'ancrage comprend au moins un élément d'ancrage distal (52) disposé à ou à proximité de ladite extrémité distale de ladite endoprothèse auto-extensible tubulaire, et ledit au moins un élément d'arrêt comprend au moins un élément d'arrêt distal (56a) qui s'étend radialement vers l'extérieur à partir dudit fil d'avancement de noyau et qui est configuré de manière à engager ledit au moins un élément d'ancrage distal lorsque ledit fil d'avancement de noyau est déplacé de façon distale longitudinalement en direction dudit au moins un élément d'ancrage distal,
**caractérisé en ce que** ledit au moins un élément d'ancrage comprend au moins un élément d'ancrage intermédiaire (46) disposé à ou à proximité de ladite partie intermédiaire de ladite endoprothèse auto-extensible tubulaire, et ledit au moins un élément d'arrêt comprend au moins un élément d'arrêt intermédiaire (50a, 50b) qui s'étend radialement vers l'extérieur à partir dudit fil d'avancement de noyau et qui est configuré de manière à engager ledit au moins un élément d'ancrage intermédiaire lorsque ledit fil d'avancement de noyau est déplacé de façon longitudinale en direction dudit au moins un élément d'ancrage intermédiaire.

2. Système selon la revendication 1, dans lequel ledit au moins un élément d'ancrage comprend au moins un élément d'ancrage proximal (40) disposé à ou à proximité de ladite extrémité proximale de ladite endoprothèse auto-extensible tubulaire, et ledit au moins un élément d'arrêt comprend au moins un élément d'arrêt proximal qui s'étend radialement vers l'extérieur à partir dudit fil d'avancement de noyau et qui est configuré de manière à engager ledit au moins un élément d'ancrage proximal lorsque ledit fil d'avancement de noyau est déplacé de façon longitudinale en direction dudit au moins un élément d'ancrage proximal.

3. Système selon la revendication 2, dans lequel ledit au moins un élément d'ancrage proximal comprend deux éléments d'ancrage proximaux (42a, 42b) disposés sur des côtés opposés dudit fil d'avancement de noyau.

4. Système selon la revendication 2, dans lequel ledit au moins un élément d'arrêt comprend des premier et second éléments d'arrêt proximaux (44a, 44b) espacés l'un de l'autre, et ledit au moins un élément d'ancrage proximal est disposé entre lesdits premier et second éléments d'arrêt proximaux.

5. Système selon la revendication 3, dans lequel ledit au moins un élément d'arrêt (38) comprend des premier et second éléments d'arrêt proximaux (44a, 44b) espacés l'un de l'autre, et lesdits deux éléments d'ancrage proximaux sont disposés entre lesdits premier et second éléments d'arrêt proximaux.

6. Système selon la revendication 1, dans lequel ledit au moins un élément d'ancrage distal comprend deux éléments d'ancrage distaux (54a, 54b) disposés sur des côtés opposés dudit fil d'avancement de noyau.

7. Système selon la revendication 1, dans lequel ledit au moins un élément d'arrêt comprend des premier et second éléments d'arrêt distaux (56a, 56b) espacés l'un de l'autre, et ledit au moins un élément d'ancrage distal (52) est disposé entre lesdits premier et second éléments d'arrêt distaux.

8. Système selon la revendication 6, dans lequel ledit au moins un élément d'arrêt comprend des premier et second éléments d'arrêt distaux (56a, 56b) espacés l'un de l'autre, et lesdits deux éléments d'ancrage distaux sont disposés entre lesdits premier et second éléments d'arrêt distaux.

9. Système selon la revendication 1, dans lequel ledit au moins un élément d'ancrage intermédiaire comprend deux éléments d'ancrage intermédiaires (48a, 48b) disposés sur des côtés opposés dudit fil d'avancement de noyau.

10. Système selon la revendication 1, dans lequel ledit au moins un élément d'arrêt comprend des premier et second éléments d'arrêt intermédiaires (50a, 50b) espacés l'un de l'autre, et ledit au moins un élément d'ancrage intermédiaire est disposé entre lesdits premier et second éléments d'arrêt intermédiaires.

11. Système selon la revendication 9, dans lequel ledit au moins un élément d'arrêt comprend des premier et second éléments d'arrêt intermédiaires (50a, 50b) espacés l'un de l'autre, et lesdits deux éléments d'ancrage intermédiaires sont disposés entre lesdits premier et second éléments d'arrêt intermédiaires.

12. Système selon la revendication 1, dans lequel ledit au moins un élément d'arrêt (44, 50, 56) présente un diamètre supérieur ou égal à une extension radiale intérieure dudit au moins un élément d'ancrage lorsque ladite endoprothèse auto-extensible tubulaire se trouve dans ladite configuration comprimée.

13. Système selon la revendication 1, dans lequel ledit au moins un élément d'arrêt (44, 50, 56) comprend un manchon attaché fixement audit fil d'avancement de noyau, une bobine attachée fixement audit fil d'avancement de noyau, ou un profil de degré meulé formé sur ledit fil d'avancement de noyau.

14. Système selon la revendication 1, dans lequel ledit au moins un élément d'arrêt (40, 50, 56) comprend une partie agrandie dudit fil d'avancement de noyau présentant un diamètre supérieur ou égal à une extension radiale intérieure dudit au moins un élément d'ancrage lorsque ladite endoprothèse auto-extensible tubulaire se trouve dans ladite configuration comprimée.
